(19) Europäisches Patentamt / European Patent Office / Office européen des brevets

(11) **EP 3 720 403 B1**

(12) **EUROPEAN PATENT SPECIFICATION**

(45) Date of publication and mention
of the grant of the patent:
**24.01.2024 Bulletin 2024/04**

(21) Application number: **18834010.3**

(22) Date of filing: **06.12.2018**

(51) International Patent Classification (IPC):
**A61F 13/49** (2006.01)        **B32B 5/12** (2006.01)
**B32B 5/26** (2006.01)        **B32B 27/12** (2006.01)
**B32B 3/00** (2006.01)

(52) Cooperative Patent Classification (CPC):
**A61F 13/49017; A61F 13/4902; B32B 3/00;**
**B32B 5/022; B32B 5/024; B32B 5/026; B32B 5/08;**
**B32B 5/12; B32B 5/26; B32B 7/12; B32B 27/12;**
A61F 2013/49025; B32B 2250/03; B32B 2250/20;
B32B 2255/02;                                    (Cont.)

(86) International application number:
**PCT/IB2018/059717**

(87) International publication number:
**WO 2019/111203 (13.06.2019 Gazette 2019/24)**

(54) **COMPOSITE LAMINATE BODY AND DISPOSABLE DIAPER**

VERBUNDSCHICHTKÖRPER UND WEGWERFWINDEL

CORPS STRATIFIÉ COMPOSITE ET COUCHE JETABLE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.12.2017   JP 2017235313
27.02.2018   JP 2018033536
30.03.2018   JP 2018068119
12.04.2018   JP 2018076763
16.04.2018   JP 2018078256
24.04.2018   JP 2018083332
25.04.2018   JP 2018083604**

(43) Date of publication of application:
**14.10.2020 Bulletin 2020/42**

(73) Proprietor: **Toray Opelontex Co., Ltd
Osaka 530-8222 (JP)**

(72) Inventors:
• **TANAKA, Toshihiro
Otsushi
Shiga 520-8558 (JP)**
• **SUZUKI, Katsuya
Otsushi
Shiga 520-8558 (JP)**
• **TAKEUCHI, Fumio
Otsushi
Shiga 520-8558 (JP)**
• **TANIGUCHI, Kouichi
Otsushi
Shiga 520-8558 (JP)**

(74) Representative: **Carpmaels & Ransford LLP
One Southampton Row
London WC1B 5HA (GB)**

(56) References cited:
**WO-A1-00/00051          WO-A1-2018/084145
JP-A- 2014 207 973       US-A1- 2002 002 021
US-A1- 2003 111 162**

(52) Cooperative Patent Classification (CPC): (Cont.)
B32B 2255/26; B32B 2262/0207; B32B 2262/0246;
B32B 2262/0253; B32B 2262/0261;
B32B 2262/0276; B32B 2262/0292; B32B 2262/04;
B32B 2262/062; B32B 2262/08; B32B 2262/14;
B32B 2307/4026; B32B 2307/51; B32B 2307/718;
B32B 2437/00; B32B 2451/00; B32B 2479/00;
B32B 2535/00; B32B 2555/02; B32B 2601/00

## Description

The present invention relates to a composite laminate body and a disposable diaper using the composite laminate body, and more particularly to a composite laminate body with elasticity that can be suitably used for applications such as clothing that contacts the skin, specifically underwear and sportswear, as well as sanitary materials like disposable diapers for children and adults, and catamenial products, where the composite laminate body has a beautiful appearance with excellent uniformity of folds in a gather part due to optimizing the shape of a cloth and elastic fibers that are used, and where the return stress upon release of an elongating load (hereinafter also referred to as unload power) has low elongation dependency, and which has a soft feel that is characteristic when wearing, and that has a wide size adaptation range, The present invention also relates to a disposable diaper that uses this composite laminate body.

BACKGROUND

Conventionally, materials with stretchability have been used in order to enhance the feel of wearing and wearing stability in applications that contact this skin such as hygienic materials like disposable diapers and the like. For example, a composite laminate body where elastic fibers are inserted between laminate nonwoven materials is used in order to enhance the fit to the body around the legs, around the waist, and around the abdomen, and the like, and to prevent leaking of urine.

These composite laminate bodies having stretchability are often produced by stretching a plurality of elastic fibers to a predetermined draft, and then laminating in this condition using a plurality of sheet-like cloths such as nonwoven materials and the like, and the configuration of the composite laminate body often has a hot melt resin extending in a direction parallel to the elastic fibers in the sheet-like cloths such as a nonwoven material or the like, and a composite laminate body with this configuration is often used in disposable diapers and usually forms a gather member (for example, refer to Patent Document 1).

Furthermore, when constructing the gather member, a plurality of elastic stretchable members (elastic fibers) are disposed between sheet-like cloths such as a nonwoven material or the like, thermal fusing fibers are provided to extend in a direction that intersects with the elastic stretchable members the elastic stretchable members are secured between the sheet-like cloths by thermally fusing the thermal fusing fibers to form a gather member (for example, refer to Patent Document 2). Furthermore, another known gather member is made by applying a hot melt adhesive in place of the thermally fusing resin in a striped pattern in a direction that intersects with the elastic stretchable members using an application method referred to as design coating that applies in a predetermined pattern in a predetermined range, and the elastic stretchable members are secured to the cloth intermittently in the longitudinal direction by the hot melt adhesive (for example, refer to Patent Document 3). Furthermore, a design coat application system and a coating method using a design roller having a plate with protrusions and recesses are also known (for example, Patent Document 4).

With a composite laminate body, and in particular, with a composite laminate body having an adhesive resin (particularly a hot melt adhesive) arranged and extending in a direction parallel to the elastic fiber, the hot melt adhesive is applied to substantially the entire region of the elastic fiber and bonds to the cloth made of nonwoven material on both sides, so the elastic fibers must stretch the cloth and the hot melt adhesive when the composite laminate body is stretched, and therefore there is a problem that the return properties are impaired when the stretching load is released, in particular. Therefore, the size adaptation range of the gather part formed by the composite laminate is narrow, and is necessary to establish a large number of sizes of products such as disposable diapers, for example. Establishing such a large number of sizes is a major burden for manufacturers. Reducing the number of sizes may impair the feel when wearing the product, such as a soft feeling when worn.

JP 2014 207973 describes an expansion structure such that: a sheet joint part is formed by joining a first sheet material and a second sheet material together by an adhesive agent intermittently arranged in the expansion direction and continuing in a direction crossing the expansion direction; elastic expansion members are fastened by the adhesive agent to at least one of the first sheet material and the second sheet material on a position crossing the sheet joint part; the first sheet material and the second sheet material contract with the contraction of the elastic expansion members so that parts positioned between the sheet joint parts in the first sheet material and the second sheet material expand oppositely to each other to form pleats respectively; and a width in the expansion direction of each of the sheet joint parts is 0.5-4 mm, and an interval of the sheet joint parts adjacent to each other is 4-8 mm.

Furthermore, an elastic composite laminate body having a gather part with folds is generally required to have uniform folds and to have a beautiful appearance and desired gather part performance. It is difficult to satisfy both the required characteristic of a beautiful appearance and the required characteristic of excellent feel when wearing, when the composite laminate body is used as a disposable diaper or the like as described above.

Patent Document 1: Japanese Unexamined Patent Application 2005-320636
Patent Document 2: Japanese Unexamined Patent Application 2013-202056

Patent Document 3: Japanese Patent Application Publication 2017-185336
Patent Document 4: Japanese Unexamined Patent Application 2014-076077

## SUMMARY OF THE INVENTION

Therefore, an object of the present invention is to provide a composite laminate body that can provide both the appearance aesthetics and functionality of the gather parts required of clothing and high-end disposable diaper products, and in particular has suitable specific performance for a disposable diaper or the like, particularly low elongation dependency of the unload power, and that has a characteristically soft feel when wearing, and that has a wide size adaptation range, and to provide a disposable diaper using this composite laminate body.

The invention is defined by the appended claims. In order to achieve the aforementioned object, the composite laminate body of the present invention is a composite laminate body, containing: a plurality of polyurethane elastic fibers arranged in parallel in one direction, interposed between two sheets of cloth; and resin for bonding the elastic fibers and the two sheets of cloth, provided in a plurality of strands arranged to be parallel in one direction that intersects with the elastic fibers; wherein the two sheets of cloth and the elastic fibers are separated between locations where adjacent resin strands and elastic fibers intersect in the longitudinal direction of the elastic fibers; the fineness of the elastic fibers is 350 dtex or less; a ratio of fineness of the elastic fibers with regard to the fineness of the fibers that compose the cloths is 0.5 or higher and 300 or lower; and when two cycles of 130% elongation and return are performed, the following Formula 1 is satisfied, where (A) is the return stress at 30% elongation in the second cycle, and (B) is the return stress at 100% elongation in the second cycle.

$$[\text{Formula 1}] \qquad (A)/(B) \times 100\% \geqq 50\%$$

The composite laminate body according to the present invention as described above has a configuration where the elastic fibers are bonded to the cloths on both sides in the longitudinal direction of in an intermittent desired form of the elastic fibers by means of resin that is applied with high precision only in the required amount in a predetermined direction in a predetermined region of the composite laminate body by so-called design coating or the like, and a structure where the two cloths and the elastic fibers are separated between locations where adjacent resin strands and elastic fibers intersect in the longitudinal direction of the elastic fibers has high regularity and provides a high level of stretchability, and therefore a composite laminate body is achieved that can provide gather parts with smooth, regular, and uniform folds that provide excellent fit and excellent feel while wearing. Furthermore, in order to enhance the appearance aesthetics of the composite laminate body having this type of gather part, in particular, in order to obtain an appearance where the presence of the elastic fibers is not noticeable, the present invention has a configuration where the ratio of fineness of the elastic fibers to the fineness of the fibers that compose the cloth is 0.5 or higher and 300 or lower. Furthermore, the present invention has a configuration where the composite laminate body satisfies the aforementioned formula 1. This configuration exhibits low elongation dependency of the return stress upon release of the elongation load, or in other words the unload power, and thereby can provide a product such as a disposable diaper using this composite laminate body that has a characteristically soft feel when wearing, and has a wide size adaptation range. Therefore, in products using this type of composite laminate body, beautiful appearance with folds with excellent uniformity in a gather part is achieved, and excellent wearing feel and a wide size adaptation range are achieved, by optimizing the form of the cloth and the elastic fibers that are used as described above.

The composite laminate body according to the present invention preferably has an elongation of 150% or more when a forward stress during the first cycle elongation of the composite laminate body is 1000 cN/5 cm width. In other words, when the composite laminate body is used for product such as disposable diapers or the like, it is desirable that elongation is possible without problems at least above a certain level of elongation (especially 150% or higher), but when it elongated for wearing, it is preferable to have the aforementioned characteristics in order to achieve a perfect feel while wearing, particularly a perfect tightening force).

Furthermore, with the composite laminate body according to the present invention, if the elastic fibers are too thick, the elastic fibers will easily be noticeable in appearance when used in the composite laminate body, and there is a possibility that the appearance aesthetics will be impaired, but if the fineness of the elastic fiber is 350 dtex (decitex) or lower, the appearance aesthetics improving affect of the present invention can be more remarkably exhibited, and the uniformity of the folds in the gather part will be enhanced. Therefore, to further enhance the effect of the present invention, the fineness of elastic fiber is 350 dtex or less.

Furthermore, in order to further increase the commercial value of the composite laminate having the gather portion, concerning the visual appearance, the composite laminate body according to the present invention preferably satisfies the requirement that the elastic fiber in the composite laminate body will not be visible, or the presence of the elastic fiber cannot be confirmed, or the elastic fibers will be very difficult to see or the presence of the elastic fiber will be very difficult to confirm. In order to satisfy these requirements, the composite laminate body of the present invention has a configuration where the ratio of the fineness of the elastic fibers to the fineness of the fibers that compose the aforementioned cloths is 0.5 or higher and 300 or lower, and when the composite laminate body is in a condition of being stretched to a maximum in the direction that the elastic fibers extend, and the color of the outer surface of the cloth is measured using a spectrocolorimeter from outside of the composite laminate body in the direction orthogonal to the elastic fibers, in the following formula using the L* value, a* value, b* value at a point where the L* value is a maximum (L*1, a*1, b*1) and L* value, a* value, b* value at a point where the L* value is a minimum L*2, a*2, b*2), the color difference variation defined by ΔE*v is 1.0 or lower.

$$\Delta E^{*}v = \sqrt{[(L^{*}1 - L^{*}2)^2 + (a^{*}1 - a^{*}2)^2 + (b^{*}1 - b^{*}2)^2]}$$

This color difference variation ΔE*v is expressed as the degree of color variation in the colorimetry direction using the above equation when the color of the outer surface of the cloth is measured from the outside of the composite laminate body using a spectrocolorimeter in the direction that intersects with the elastic fibers, in a condition where the composite laminate body is stretched to a maximum in the direction that the elastic fibers extend. If the cloth and the elastic fiber have the same color, the color difference variation ΔE*v is particularly dependent on the degree of change in the L*value that indicates lightness, thereby making it difficult to see the elasticity of the elastic fiber, and the ease or difficulty of confirming the presence thereof is controlled thereby. As the color difference variation ΔE*v increases, the change in the color difference becomes larger at the regions that intersect with the elastic fibers, and therefore with regard to the appearance, the elastic fibers will not easily be seen, and the presence of the elastic fibers cannot easily be confirmed. If the color difference variation ΔE*v is 1.0 or less, the elastic fibers will be difficult to see, or the presence of the elastic fibers will not be able to be confirmed, or the elastic fibers will be extremely difficult to see or the presence thereof will be extremely difficult to confirm, and thus the required property is satisfied. Therefore, a composite laminate body is achieved that the desired stretchability performance, and has folds with favorable regularity with excellent fitness while wearing and smooth feel on the skin, and in particular, the presence of elastic fibers when viewed from the outside in particular will not be conspicuous, and the uniform appearance will be aesthetically excellent.

Furthermore, with the composite laminate body of the present invention, the thickness of the composite laminate body at a location where the resin that extends in one direction intersects with the elastic fibers is preferably 0.1 mm or larger and 5.0 mm or smaller, and more preferably 0.1 mm or larger and 2.0 mm or smaller.

Furthermore, with the composite laminate body of the present invention, the maximum value for the thickness of the composite laminate body between locations where the elastic fibers intersect with the resin that extends in one direction, and that is adjacent in the longitudinal direction to the elastic fibers is preferably 1 mm or larger and 20 mm or smaller.

Furthermore, with the composite laminate body of the present invention, the width of one strand of resin that extends in one direction is 0.2 mm or more and 10 mm or less, in a condition where the composite laminate body is stretched to the maximum in the extending direction of the elastic fibers. This preferable resident width can be precisely adjusted using a design coater.

Furthermore, with the composite laminate body of the present invention, interval that extends in one direction is 1 mm or more and 20 mm or less, in a condition where the composite laminate body is stretched to the maximum in the extending direction of the elastic fibers.

Furthermore, in the composite laminate body of the present invention, the heat softening point of the elastic fiber is preferably 100°C or more and 240°C or less. Therefore bonding to the resin that was design coated can easily be performed.

Furthermore, with the composite laminate body of the present invention, the plurality of resin strands that intersect with the elastic fibers in one direction preferably includes the same component as a constituent component of the cloth and/or elastic fibers. Therefore bonding to the resin that was design coated can be simplified.

The present invention provides a product that uses the composite laminate body according to the present invention as described above, and in particular, a disposable diaper that uses the composite laminate body according to the present invention as described above as a gather part.

In this manner, the composite laminate body according to the present invention can provide a composite laminate body that has both the appearance aesthetics and functionality of the gather part required of clothing and high-end disposable diaper products, and in particular has suitable specific performance for a disposable diaper or the like, particularly low elongation dependency of the unload power, and that has a characteristically soft feel when wearing, and that has a wide size adaptation range, and can provide a disposable diaper using this composite laminate body.

BRIEF DESCRIPTION OF THE DRAWINGS

FIG. 1 is an appearance photograph of an example of a test piece of a composite laminate body according to the present invention, taken from a substantially planar direction.

FIG. 2 is an appearance photograph of a test piece of the composite laminate body of FIG. 1, taken from a substantially cross-sectional direction, where A represents the thickness at a position where the elastic fiber intersects with the resin that extends in one direction, and B indicates the measurement location of the maximum value of the thickness between locations where the elastic fiber intersects with the resin that extends in one direction.

FIG. 3 is an appearance photograph of the test piece of the composite laminate body of FIG. 1 taken from a substantially planar direction, showing a condition of partial elongation when the elastic fiber is elongated by hand in the extending direction.

FIG. 4 is an appearance photograph of a test piece of the composite laminate body of FIG. 3 taken from a substantially planar direction, showing a condition of maximum extension in the same direction.

FIG. 5(A)-(D) is a schematic plan view illustrating a sampling method of a measurement sample of a composite laminate body according to the present invention.

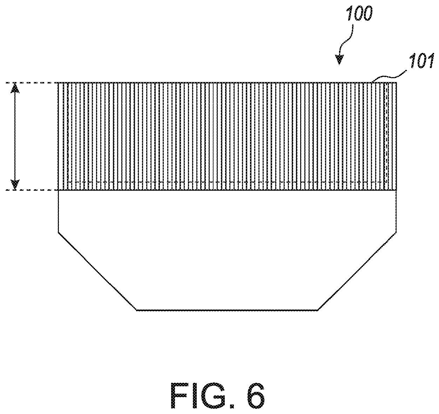

FIG. 6 is a schematic front view of a prototype of a disposable diaper that uses the composite laminate body according to the present invention.

DESCRIPTION

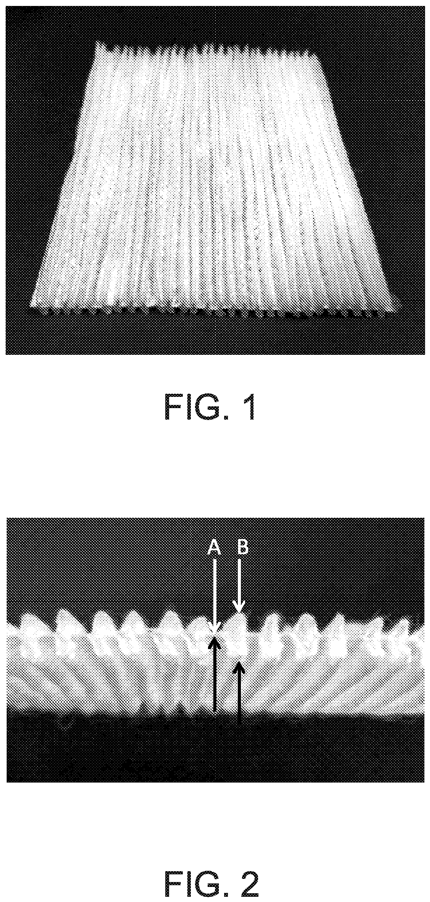

A preferred embodiment of the present invention is described below in detail using FIG. 1.

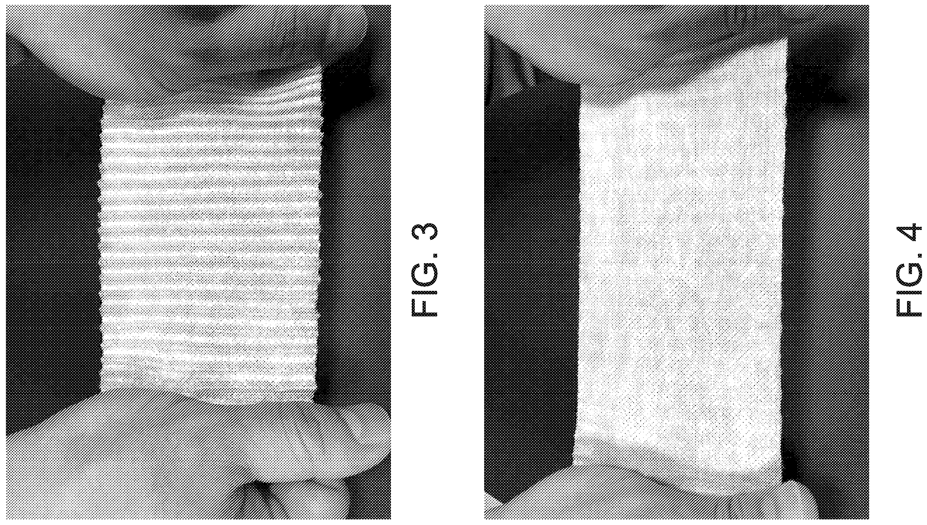

First, the form of the composite laminate body obtained by the present invention will be described. FIG. 1 shows an example of an appearance photograph of a representative example of a test piece of a composite laminate body manufactured by the present invention, taken from a substantially planar direction. FIG. 2 shows an example of an appearance photograph of a test piece of the composite laminate body of FIG. 1, taken from a substantially cross-sectional direction, where A in the figure represents the thickness at a position where the elastic fiber intersects with the resin that extends in one direction, and B indicates the measurement location of the maximum value of the thickness between locations where the elastic fiber intersects with the resin that extends in one direction. FIG. 3 shows an example of an appearance photograph of the test piece of the composite laminate body of FIG. 1 taken from a substantially planar direction, showing a condition of partial elongation when the elastic fiber is elongated by hand in the extending direction. FIG. 4 shows an example of an appearance photograph of a test piece of the composite laminate body of FIG. 3 taken from a substantially planar direction, showing a condition of maximum extension in the same direction.

In the composite laminate body of the present invention, a plurality of elastic fibers made from polyurethane elastic thread, for example, are arranged in parallel in one direction between two cloths. The elastic fibers are arranged in a straight line or curved line or a combination thereof, and are sandwiched between the cloths.

In the composite laminate body of the present invention, a plurality of resins strands are arranged in a direction intersecting with the elastic fibers and extend in one direction. In other words, the resin strands are arranged in a direction that intersects the direction in which the elastic fiber is inserted. The resin is a resin for joining two sheets of cloth and elastic fiber, which can be a resin for bonding that is applied with high precision in only the necessary amount in a predetermined region in a predetermined direction by so-called design coating or the like, or can be a resin but for bonding produced from the cloth itself or the elastic fiber itself (in particular the cloth itself), using ultrasonic waves or heating. The 2 clothes and the elastic fibers are spaced apart from each other between the locations where the elastic fiber and the resin that are adjacent to each other intersect in the longitudinal direction of the elastic fiber. In the foregoing, extending in one direction means that the resin strands are linear and are arranged in one direction as a whole. The linear form may be either a straight line shape or a curved shape or a combination thereof. Arranged in one direction as a whole means to be arranged such that a linear form falls within a range of parallel lines with a width of 10 mm, and the direction of the one direction is in the direction of the virtual parallel lines. Although the angle at which the elastic fiber and the resin extending in one direction intersect is not particularly limited, it is preferably within a range of 90 $\pm$ 20°, more preferably in a range of 90 $\pm$ 10°, and even more preferably within a range 90 $\pm$ 5°.

In the new composite laminate body of the present invention, each cloth and the elastic fibers are spaced apart from each other between the locations where the elastic fiber and the resin that extends in one direction intersect and that are adjacent to each other in the longitudinal direction of the elastic fiber.

The thickness between the locations where the elastic fiber intersects with the resin that extends in one direction is preferably 0.1 mm or more and 2.0 mm or less, more preferably 0.2 mm or more and 2.0 mm or less. If the thickness is less than 0.1 mm, the presence of the elastic fibers in the composite laminate body might be visually distinguishable, and when the composite laminate body is elongated, the presence of the elastic fibers will be more readily distinguishable, soul the appearance anesthetics of the composite laminate body may be inferior. If the thickness is larger than 2.0 mm, the folds that are formed might easily buckle, and the sense of folds with favorable regularity that are smoothed and provide a sense of fitting while wearing might be unsatisfactory.

Furthermore, the maximum value for the thickness between locations that are adjacent in the longitudinal direction of the elastic fiber is preferably 1 mm or more and 20 mm or less, more preferably 2 mm or more and 10 mm or less. If the thickness is less than 1 mm, the presence of the elastic fibers in the elongated composite laminate body might clearly be visually distinguishable, and when the composite laminate body is elongated, the presence of the elastic fibers will be more readily distinguishable, soul the appearance anesthetics of the composite laminate body may be inferior. If the thickness is larger than 20 mm, the folds that are formed might easily buckle, and the sense of folds with favorable regularity that are smoothed and provide a sense of fitting while wearing might be unsatisfactory.

The width of the resin that extends in one direction is preferably 0.2 mm or more and 10 mm or less when extended to a maximum, more preferably 0.4 mm or more and 6 mm or less. If the width of the resin is less than 0.2 mm, the elastic fibers in the composite laminate body will easily move, and if stretching is repeatedly performed, the uniformity of the folds me deteriorate, adjacent elastic fibers will become closer, the presence there of may be visually distinguishable, and thus the appearance anesthetics of the composite laminate body might be inferior. If the width of the resin is more than 10 mm, the presence of the elastic fibers may easily be noticeable as streaks or stripes, when the composite laminate body is observed with transmitted light. Furthermore, elongation of the composite laminate body might decrease and replacement dimensions might increase, or the feeling of folds with favorable uniformity that are smooth and fitting while wearing might be unsatisfactory.

Furthermore, the interval between the resin strands that extend in one direction is preferably 1 mm or more and 20 mm or less, when extended to a maximum of the composite laminate body, more preferably 2 mm or more and 15 mm or less. The interval between the resin strands that extend in one direction refers to the distance between the centers of the resin strands that extend in one direction. If this interval is less than 1 mm, the presence of the elastic fibers in the composite laminate body might clearly be visually distinguishable, and if the composite laminate body is stretched, the presence will be even more pronounced, and thus the appearance anesthetics of the composite laminate body might be inferior. If the thickness is larger than 20 mm, the folds that are formed might easily buckle, and the sense of folds with favorable regularity that are smoothed and provide a sense of fitting while wearing might be unsatisfactory.

The cloths that are used in the composite laminate body of the present invention are preferably woven fabrics, knitted fabrics, or nonwoven fabrics, but a nonwoven fabric is particularly preferable, and the nonwoven material can be obtained by a wet nonwoven material manufacturing method such as a papermaking method or the like, or by a dry nonwoven material manufacturing method such as a resin bond method, a thermal bond method, a needle punch method, a spunbond method, a spun lace method, a melt blow method, or a flash spinning method. The basis weight of the nonwoven fabric is preferably 10 to 20 g/m2 or less, and more preferably 12 to 18 g/m2.

The material of the fibers constituting the fabric is not particularly limited, but it is preferable to use synthetic fibers such as polyester, polyamide, polyacrylonitrile, polypropylene, polyethylene, copolymers of various $\alpha$-olefins such as propylene and ethylene, and polyurethane, regenerated fibers such as rayon and acetate, or the like, semisynthetic fibers, or natural fibers such as wool, cotton, and the like.

The form of the fibers constituting the cloth may be either a long fiber filament or a short fiber spun yarn, and may be lightly selected from a blend of two or more types of fibers, mixed fibers, crimped fibers, or a composite fiber or the like.

In the composite laminate body of the present invention, elastic fibers are used in at least a part.

The elastic fiber used in the present invention can be a crimped fiber. The elastic fiber used in the present invention is a polyurethane elastic fiber (typically, the polyurethane elastic yarn as described above), from the perspective that the stretchability of the composite laminate body will be thoroughly demonstrated.

The elastic fibers used in the composite laminate body of the present invention may be bare yarn, or may be coated (covered) with other elastic fibers or non-elastic fibers. Bare yarn is most preferable from the perspective of the stretchability of the composite laminate body.

Note that the polyurethane elastic fiber is preferably composed of a long-chain diol such as copolyester diol as a soft segment, a diisocyanate such as diphenylmethane-4,4 diisocyanate as a hard segment, and a bifunctional hydrogen compound as a chain extender.

With the present invention, polyurethane elastic fiber is used as described above from the perspective of providing the desired stretchability to the final product.

The polyurethane polymer that is used in the polyurethane elastic fibers that can be used in the present invention preferably contains a soft segment primarily containing longchain polyether segment, polyester segment or polyether ester segment, or the like, and a hard segment primarily containing an isocyanate, and a diamine or a diol which are chain extenders.

The raw materials constituting the soft segment of the polyurethane polymer can be: 1) a polymer or copolymer obtained from tetrahydrofuran (hereinafter abbreviated as THF), tetramethylene glycol, 3-methyl-1,5-pentanediol, 2,2-dimethyl-1,3-propanediol, 3-methyltetrahydrofuran (hereinafter abbreviated as 3MeTHF) or the like 2) a polyester segment obtained from a diol such as ethylene glycol, tetramethylene glycol, 2,2-dimethyl-1,3-propanediol and a dibasic acid such as adipic acid, succinic acid, and the like, 3) a poly-(pentane-1,5-carbonate) diol, poly-(hexane-1,6-carbonate) diol, and the like, but PTMG obtained from tetramethylene glycol is preferable. More preferable is a polyether segment which is a copolymer obtained from 2,2-dimethyl-1,3-propanediol and 3MeTHF.

With the elastic fiber used in the composite laminate body of the present invention, the polyurethane polymer is obtained by reacting a prepolymer product obtained by polyaddition reaction of the hydroxyl terminal soft segment precursor with organic diisocyanate (capping reaction) using a diamine chain extender or diol or obtained by chain elongation using a chain extender. Furthermore, it is also preferable to further react the prepolymer product with an organic diisocyanate and then cause to react with a chain extender.

Examples of the organic diisocyanate to be used for the polyurethane polymer in the present invention include MDI, tolylene diisocyanate (TDI), bis-(4-isocyanatocyclohexyl)-methane (PICM), hexamethylene diisocyanate, 3,3,5-trimethyl-5-methylenecyclohexyl diisocyanate, and the like.

Various diamines such as ethylenediamine, 1,3-cyclohexanediamine, 1,4-cyclohexanediamine and the like are preferably used as diamine chain extenders for forming polyurethane urea.

The diamine chain extender is not limited to only one kind of diamine but may be composed of a plurality of types of diamines. A chain terminator can be included in the reaction mixture to aid in the adjustment of the final molecular weight of the polyurethane urea. Usually, a monofunctional compound having an active hydrogen as a chain stopper, such as diethylamine, can be used.

Furthermore, the chain extender is not limited to the aforementioned diamines, but can also be a diol. In particular, this is suitable for obtaining elastic fibers having a thermal softening point of 100°C. to 180°C. Examples thereof include ethylene glycol, 1,3-propanediol, 1,4-butanediol, neopentyl glycol, 1,2-propylene glycol, 1,4-cyclohexanedimethanol, 1,4-cyclohexanediol, 1,4-bis (β-hydroxyethoxy) benzene, bis (β-hydroxyethyl) terephthalate, paraxylylene diol and the like. The diol chain extender is not limited to only one kind of diol but may be composed of a plurality of types of diols. Furthermore, a compound containing one hydroxyl group that reacts with an isocyanate group can be used in combination. In this case, the method to obtain the polyurethane can be any type of method such as a melt polymerization method, solution polymerization method, and the like. Regarding the formulation for polymerization, there is no particular limitation, and a method of synthesizing the polyurethane by simultaneously reacting, for example, a polyol, a diisocyanate, and a chain extender made of a diol can be adopted, and any method may be used.

Further, it is also preferable to incorporate a stabilizer, a thermal conductivity improver, and a pigment within a range that does not impair the effect of the present invention.

For example, a light stabilizer, antioxidant, or the like can be added, such as so-called BHT, a hindered phenolic agent such as a "Sumilizer (registered trademark) GA-80" or the like manufactured by Sumitomo Chemical Co., Ltd., a benzotriazole such as "Tinuvin (registered trademark) manufactured by BASF, benzophenone agents, phosphorus agents, various hindered amine agents, a silicone resin powder or a fluorine resin powder with a polyvinylidene fluoride group or the like, metal soap such as magnesium stearate, disinfectants including silver, zinc, and compounds thereof, deodorants, lubricants such as silicones and mineral oils, various kinds of antistatic agents such as barium sulfate, cerium oxide, betaine, phosphate compounds, phosphate ester compounds, and the like, or these may be present by reacting with a polymer. Furthermore, in order to further enhance the durability to light and various kinds of nitric oxide in particular, a nitrogen oxide capturing agent such as HN-150 manufactured by Japan Hydrazine Co., Ltd., "Hostanox (registered trademark)" SE 10 manufactured by Clariant Corporation, and the like, or a thermal oxidation stabilizer or the like are preferably contained.

Alumina, boron nitride, aluminum nitride, silica, silicon nitride, magnesium oxide, magnesium carbonate, silicon carbide or the like are preferably added as a thermal conductivity improver in order to promote melting and heat softening.

For example, titanium oxide, zinc oxide, zirconium phosphate or the like is preferably added as a pigment. Of these, titanium oxide is preferable from the viewpoint of suppressing glare due to stripping of the elastic fiber and obtaining a composite laminate body having a homogeneous appearance in which the elastic fiber is inconspicuous. Rutile type or anatase type are preferably used for the case of titanium oxide. The average primary particle diameter is preferably in the range of 0.15 $\mu$m to 0.3 $\mu$m, from the viewpoint of suppressing reflection of light and stably producing a polyurethane elastic yarn. Furthermore, the amount in the polyurethane type elastic fiber is preferably 0.3% or more by mass, from the viewpoint of prevention of glare, and 3% by mass or less, from the viewpoint of preventing clogging of the spinneret and the like and stably spinning the polyurethane type elastic fiber.

Alumina, boron nitride, aluminum nitride, silica, silicon nitride, magnesium oxide, magnesium carbonate, silicon carbide or the like are preferably added as a thermal conductivity improver in order to promote melting and heat softening.

N,N-dimethylacetamide (hereinafter abbreviated as DMAc), dimethylformamide, dimethylsulfoxide, N-methylpyrrolidone and the like can be used as a solvent when the polyurethane polymer is made into a solution, but DMAc is the most commonly used solvent.

A dry spinning method of obtaining a polyurethane elastic filament yarn is preferable, at a solution concentration for the polyurethane polymer of 30 to 50% by mass (based on the total mass of the solution).

With the present invention, the method of spinning the polyurethane elastic fiber from the polyurethane polymer is not particularly limited, and for example, 1) a melt spinning method, dry spinning method, wet spinning method, or the like can be adopted as a spinning method for a polyurethane elastic fiber using a diol as a chain extender. Furthermore, 2) a dry spinning method can usually be adopted as a spinning method for polyurethane elastic fibers using a diamine as a chain extender.

Furthermore, with the present invention, the use of filament yarn of polyurethane elastic fiber is preferable from the viewpoint of high stretchability, particularly stretch recovery stress, but the elastic fiber itself tends to be conspicuous. Therefore, fiber specifications and combinations of the following forms are preferable.

The fineness of the elastic fibers and the fineness of the fibers constituting the cloths can be appropriately selected according to the intended use, but is preferably in a range of 0.1 to 5000 dtex.

The fineness of the fabric constituting the composite laminate in the present invention represents the maximum fineness of the fiber distributed on the surface of the fabric.

Furthermore, the fineness of the fibers constituting the fabric is preferably from 0.1 to 500 dtex, more preferably from 0.1 to 50 dtex, and most preferably from 0.3 to 30 dtex, from the viewpoint of forming folds with high uniformity. Furthermore, the fineness is preferably 0.2 to 5 dtex, and most preferably 0.2 to 2.0 dtex, from the viewpoint of feel in the composite laminate body.

Furthermore, the fineness of the elastic fiber is 350 dtex or less, and preferably 10 to 350 dtex.

When elastic fiber having an elastic fiber fineness of less than 1 dtex is used, there is a tendency that the elastic fiber and not withstand running friction during running, so yarn breakage tends to occur, and when elastic fiber exceeding 3000 dtex is used, there is a tendency that the sensor side can not endure breakage due to running friction during manufacturing.

With the composite laminate of the present invention, the ratio of the fineness of the elastic fiber to the maximum fineness of the fiber distributed on the surface of the fabric must be 0.5 or more and 300 or less, from the viewpoint of appearance quality.

In order to obtain a composite laminate having a form in which the presence of the elastic fiber in the composite laminate is difficult to discriminate, or in other words, a composite laminate having a homogeneous appearance in which the elastic fiber is inconspicuous, the influence of the surface reflected light is more dominant than the transmitted light, and the fineness ratio between the elastic fibers and the maximum fineness of the fibers distributed on the surface is important.

If the ratio of the fineness of the elastic fiber to the maximum fineness of the fiber distributed on the surface of the cloth exceeds 300, the presence of the elastic fibers are very noticeable, and the appearance quality is impaired.

If the ratio of the fineness of the elastic fiber to the maximum fineness of the fiber distributed on the surface of the cloth is 0.5 or higher and 300 or lower, the presence of the elastic fibers will not easily be visually observed, and the appearance quality will be particularly excellent.

The effect appears to be more remarkable when the maximum fineness of the fiber distributed on the surface of the fabric is within a range of 0.2 to 2.2 dtex, and more remarkable when the maximum fineness is 0.5 to 2.0 dtex. If the ratio of the fineness of the elastic fibers with regard to the maximum fineness of the fibers distributed on the surface of the cloth is larger than 300, the presence of the elastic fibers in the composite laminate body might clearly be visually distinguishable, and if the composite laminate body is stretched, the presence will be even more easily distinguished visually, and thus the appearance anesthetics of the composite laminate body will be inferior. If the fineness ratio is less than 0.5, the elasticity of the elastic fibers will be substantially insufficient, folds will not easily form, and stretchability will not be demonstrated.

Furthermore, with the method of the present invention, the color difference variation ΔE*v defined as described above is preferably a specific value or lower (1.0 or lower), in order to obtain a composite laminate body having a homogeneous appearance in which the elastic fiber is not visible or is inconspicuous. The color difference variation ΔE*v is a value calculated from the values of the L* value, the a* value, and the b* value in the Lab color specification system by a non-contact type spectrocolorimeter, which will be described later, using an equation described later. Note that the L* value in the Lab color specification system is an index representing lightness, the a* value is a position between red and green, and the b* value is an index indicating the position between yellow and blue. As the color difference variation ΔE*v increases, the change in the color difference becomes larger at the regions that intersect with the elastic fibers, and therefore with regard to the appearance, the elastic fibers will not easily be seen, and the presence of the elastic fibers cannot easily be confirmed. If the color difference variation ΔE*v is 1.0 or less, the elastic fibers will be difficult to see, or the presence of the elastic fibers will not be able to be confirmed, or the elastic fibers will be extremely difficult to see or the presence thereof will be extremely difficult to confirm, and thus the required property is satisfied.

Note that in the present invention, the elastic fiber may be original drawn yarn, and the cloth and the fibers constituting the cloth may be preliminarily colored. The method of coloring the cloth and the fibers constituting the cloth is not particularly limited, but from the viewpoint of coloring the same color as the elastic fiber, it is also preferable to color by cheese dyeing or the like, that is capable of adjusting color.

The elastic fibers used in the present invention preferably have a thermal softening point in the range of 100°C or higher and 240°C or lower, from the viewpoint of obtaining fibers which do not have practical problems including process permeability and which are superior in form at the locations where the resin extending in one direction and elastic fibers intersect with each other. When the heat softening point is lower than 100°C, the form may be broken due to the effect of heat from the resin that extends in one direction when drying in a tumbler or when manufacturing the composite laminate body, and when the softening point is above 240°C, the compatibility between the resin that extends in one direction and the elastic fiber will be low, and there may be a negative effect on the formation of folds. The range of the thermal softening point is more preferably 110°C or more and 200°C or less, and most preferably 120°C or more and 160°C or less. This range is preferable because the elastic fiber and/or the material that constitutes the cloth can be made to soften and fuse to the plurality of resin strands that extend in one direction that intersects with the elastic fiber using a known method such as a hot roller, an ultrasonic welder, a high frequency welder, an electromagnetic induction welder, or a composite welder thereof.

A plurality of strands of resin that extend in one direction that intersects with the elastic fibers are present in the cloth used in the composite laminate body of the present invention, and the resin is a resin can be any type of adhesive such as a hot melt adhesive, a solvent based adhesive, or the like, or the elastic fibers and/or the materials that constitute the cloth are softened or fused.

In the case where the material constituting the fabric is heat-softened or fused, various well-known methods such as thermal embossing and ultrasonic fusing can be adopted.

It is also preferable that a mechanical entangling method such as needle punching or water jets or the like is applied to the region of the resin extending in one direction.

Furthermore, in order to enhance the effect of the present invention, the resin preferably used as the resin extending in one direction is a resin containing the same kind of material as the cloth and the elastic fiber, and more preferably contains a cloth or a component of the elastic fiber. When such a cloth or a resin containing a component of the elastic fiber is used as the resin extending in one direction, it is also preferable to heat-soften or melt the cloth or the elastic fiber to form a resin extending in one direction, and most preferable is the case where both the fabric and the elastic fibers are formed by heat softening or melting.

The composite laminate body of the present invention is a composite laminate body having stretchability, and preferably is a garment that closely adheres to the skin, more specifically, underwear, sportswear or disposable diapers for children and adults, catamenial products, masks, medical wear, sanitary materials such as surgical garments, bandages, supporters, medical materials, and the like. Furthermore, since the composite laminate body has excellent aesthetic properties, it can also be applied to interior goods such as curtains, furniture, beddings, linings, girdles, brassieres, intimate goods, waistbands for clothing, stretchable sports wear, stretchable outer wear, and the like. Of these, the composite laminate body of the present invention is particularly suitable for use in disposable diapers.

EXAMPLES

Evaluation of the composite laminate body of the present invention is described below using examples. First, the measurement and evaluation methods for each characteristic used in the description of the present invention will be described.

Softening point

The thermal softening point was measured as one index of heat resistance of the elastic fiber. The temperature dispersion of the dynamic storage elastic modulus E' was measured for the elastic fiber, at a heating rate of 10°C/min using a dynamic modulus measuring device RSA II manufactured by Rheometrics Co., Ltd. The heat softening point was determined from the intersection of the tangent line in the plateau region of the E' curve, and the tangent line of the E' curve where E' drops due to heat softening. Note that E' is a logarithmic axis and temperature is a linear axis.

Cloth fineness

Observing the fibers on the surface of the cloth using a scanning electron microscope, the maximum value ($\alpha$) for the diameter of 10 randomly selected surface fibers was measured and the density ($\rho$) g/m3 of the material constituting the cloth was determined using the following formula.

$$\text{Fineness (dtex)} = \rho \times \pi \times (\alpha/2)^2 \times 10000$$

Fineness of elastic fiber

In the present invention, the fineness of the elastic fiber is the apparent fineness measured in accordance with ISO 2060, and the measuring method is as follows. A sample of elastic fiber to be used for measurement of the apparent fineness was used after standing for 24 hours in an environment of 20°C and 65% relative humidity. The elastic fiber was cut under a no-load condition to a length d (units: m) and the apparent fineness (dtex) was determined from the thread mass (g) of length d(m) × 10000 ÷ d, to one decimal place. Here, the length d can usually be usually 0.1 m, but it is not necessary to be one continuous fiber, and the total length d' of a plurality of fibers may be 0.1 m. In this case, when taking out a sample of the elastic fiber from the composite laminate body, it is only necessary to sample the elastic fiber arranged between the two cloths from a location away from each cloth. For example, the composite laminate body was cut along a resin strand extending in one direction that intersects with the elastic fiber using a pair of scissors, and elastic fiber pieces having a good linear shape and a total length of 0.1 ± 0.01 m were measured for dimensions using an optical microscope to select a plurality of elastic fiber pieces, and obtain the total length d'. Next, the total mass of the plurality of selected elastic fiber pieces were measured by a precision balance, and the total mass (g) × 10000 ÷ d' of the elastic fiber pieces was calculated to determine the fineness of the elastic fiber.

Measurement of color difference variation of composite laminate body

The composite laminate body to be measured stretched to a maximum and fixed in the direction of extension, and then placed on the board with a black background. Using a Color master (D 25 DP-9000 type signal processor) as a non-contact type spectrocolorimeter, the L* value, the a* value, and the b* value in the L*a*b* color system were measured using the colorimetric diameter $\varphi$ = 1 mm. The values were measured at 40 points at intervals of 0.5 mm in the width direction of the composite laminate body. From the measurement results, the color difference variation "ΔE*v" was calculated at the points with the maximum value of L* as L*1, a* 1, b*1, and at the points with the minimum value of L* as L*2, a*2, and b*2, using the following formula.

$$\Delta E^*v = \sqrt{[(L^*1 - L^*2)^2 + (a^*1 - a^*2)^2 + (b^*1 - b^*2)^2]}$$

Sampling of measurement sample and stress measurement during elongation of the sample

(1) Sampling of the measurement sample

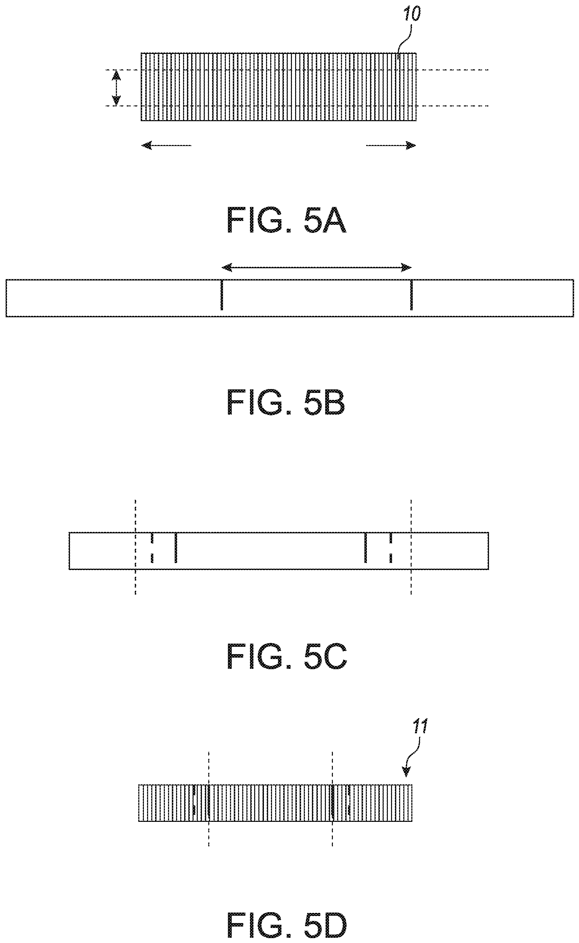

First, as illustrated in FIG. 5(A), the composite laminate body 10 is cut to a width of 5 cm in the MD direction (machine direction, in other words the direction of manufacturing a strip of the composite laminate body 10). Next, as illustrated in FIG. 5(B), the cut composite laminate body is pulled to a state without looseness, and marks are made at intervals of 20 cm in the MD direction. Next, as illustrated in FIG. 5(C), the composite laminate body is cut at a position on the outside of the aforementioned marks and is fastened by stapling at the position on the outside of the mark. This stapler fastening prevents fraying of the elastic fibers. Next, as illustrated in FIG. 5(D), the composite laminate body is relaxed in the MD direction, the link between the marks is measured and this length is used as the test length.

(2) Stress measurement during elongation

This is measured using an Instron tensile tester. The length between marks of the composite laminate body collected in the aforementioned (1) is taken as the test length. Two cycles of 130% repetitive elongation were performed, and the forward stress and return stress during elongation, and the elongation at that time were measured. The speed of the repetitive elongation was 500 mm/minute.

Fabrication of a disposable diaper prototype

A commercially available way to gather part of an adult disposable diaper was cut, and the composite laminate body is prepared in Examples 1 to 5 and Comparative Examples 1 to 4 were sown with cotton thread to fabricate a prototype 100 of a disposable diaper where the gather part 101 has a tubular shape with a width of 12 cm and extends in the waist surrounding region.

Fitness: Size Adaptability of Disposable Diaper

A prototype of a disposable diaper having the aforementioned gather part was fabricated in 3 sizes in the waist circumference (3 sizes S: 40 cm, M: 45 cm, L: 50 cm). Prototypes of disposable diapers fabricated with a tubular width of 12 cm where the stretching direction is the direction where the waist part extends, and these prototypes were evaluated by 10 judges on the feel of wearing. The waist size of the subject at this time was 80 cm $\pm$ 5 cm. The judgment results are shown by the following criteria.

◎: Comfortable feel with perfect size
○: Feel with perfect size
△: Size feels slightly small or slightly large
✕: Size is different and feel is uncomfortable

Evaluation of appearance shape of folds of composite laminate body (judgment of appearance of fold uniformity)

The composite laminate body was stretched 20% in the MD direction, fixed at both ends at a length of 30 cm, left for 24 hours at a temperature of 20°C and the relative humidity of 65%, and then the shape of the folds was visually observed and the following judgments were made.

Evaluation of folds

◎: 0 disturbances in the folds
○: 1 to 3 disturbances in the folds
△: 4 to 10 disturbances in the folds
✕: 10 or more disturbances in the folds

Example 1

As illustrated in Table 1, 18 strands of 156 dtex elastic polyurethane yarn (heat softening point: 220°C) as elastic fibers were inserted at a density of 6 g/m2 at a draft of 3.5 times during insertion between the cloths. A nonwoven material having a three layer laminate structure (referred to as "SMS" in Table 1), namely a spunbond layer/melt blow layer/spunbond layer with polypropylene (PP) spunbond nonwoven material was used as the composite laminate body with a surface fiber fineness of 1.5 dtex and a basis weight of 17 g/m2, the aforementioned polyurethane elastic yarn was inserted between two cloths, a resin for bonding was applied by design coating using a design roller (application width per strand of resin that extends in one direction, in other words hot melt resin, is 1 mm, and the interval between the resin strands that extend in one direction is 6 mm), and the elastic fibers and the cloths were intermittently bonded. The ratio of the fineness of the elastic fiber to the finest of the fiber constituting the cloths in the present invention was 104. A rubber-based hot melt adhesive (manufacturer: Bostic Nitta Co., Ltd., product number: AFX-162) was used a coating amount of 20 g/m2 as a resin for bonding the elastic polyurethane yarn and the nonwoven material as the cloth. As illustrated by the evaluation results of Table 1, wherein the color difference variation $\Delta E^*v$ is 0.4 and less than 1.0, where (A) the return stress at 30% elongation of the second cycle of the repetitive elongation cycle is 44 cN, and (B) the return stress during 100% elongation of the second cycle is 68 cN, and the value of (A)/(B)x100% in [Formula 1] is 65%, larger than 50%, and therefore the conditions meet all of the targets of the present invention. Furthermore, the fitness as the size adaptability evaluation of a disposable diaper was judged as ◎ for all three sizes S, M, and L. Furthermore, the appearance judgment of fold uniformity was evaluated as ◎ as the appearance shape evaluation of the folds of the composite laminate body. The results show that a composite laminate body can be obtained with beautiful folds having high uniformity and regularity, beautiful aesthetic appearance, excellent fit when used as a disposable diaper, low elongation dependency of the mode power, characteristically soft feel while wearing, and having a wide range of size adaptation.

Examples 2 through 5 and Comparative Examples 1 through 4

As shown in Table 1, various conditions were changed as compared to Example 1 (the heat softening point of the polyurethane elastic yarn was 220°C in all cases). Note that the term "comb gun" in Table 1 refers to a method of forming the composite laminate body by continuously and directly applying a hot melt resin to the elastic fibers using a standard comb gun, and laminating the elastic fibers and the cloths using the hot melt resin. During design coating, a composite laminate body is formed with a resin arranged and extending in a direction orthogonal to the elastic fibers, whereas with a comb gun, a composite laminate body having strands of resin arranged and extending in a direction that is parallel to the elastic fibers.

As shown in Table 1, Examples 1 to 5 satisfy the conditions set forth in the present invention, and therefore can provide a composite laminate body with folds that have high regularity and uniformity with little disturbances, with an appearance having excellent aesthetics where the presence of the elastic fibers is not noticeable, and that has excellent fit properties, feel while wearing, and adaptability to a wide size range. On the other hand, Comparative Examples 1 to 4 did not satisfy all of the conditions set forth in the present invention, and therefore could not provide a composite laminate body with aesthetics where the presence of the elastic fibers was inconspicuous, where the target performance of the folds were satisfied, and where the fit properties and adaptability across a large size range are excellent when used as a disposable diaper. In other words, Comparative Examples 1 and 2 in particular both had elongation that was insufficient and could not be measured for both the return stress during 30% elongation in the second cycle, and (B) return stress during 100% elongation in the second cycle. Therefore, both fitness and uniformity of folds were inferior. In Comparative Examples 3 and 4, the ratio of fineness of the elastic fibers with regard the fineness of the fibers that compose the cloths in particular was too large, the color difference variation ∆E*v exceeded 1.0, and the elastic fibers were noticeable, so excellent appearance aesthetics could not be obtained and the fitness when evaluated as a disposable diaper was unacceptable for some sizes.

Table 1

| | Elastic fibers (polyurethane elastic yarn) | | | | Cloths (nonwoven material) | | | Composite laminate body | | Properties of composite laminate body | | | | | Fitness | | | Fold uniformity |
|---|---|---|---|---|---|---|---|---|---|---|---|---|---|---|---|---|---|---|
| | Fineness (dtex) | No. inserted (strands) | Basis weight (g/m²) | Draft during insertion (times) | Type | Surface fiber fineness | Basis weight (g/m²) | Manufacturing method | Fineness ratio | Color difference variation $\Delta E^*v$ | (A) return stress at 30% elongation (cN) | (B) Return stress at 100% elongation (cN) | (A)/(B) x 100% | S Judgment | M Judgment | L Judgment | Appearance judgment |
| Example 1 | 156 | 18 | 6 | 3.5 | PP spunbond SMS | 1.5 | 17 | Design roller | 104 | 0.4 | 44 | 68 | 65 | ◎ | ◎ | ◎ | ◎ |
| Example 2 | 156 | 12 | 4 | 3.5 | PP spunbond SMS | 1.5 | 17 | Design roller | 104 | 0.5 | 36 | 64 | 56 | ◎ | ◎ | ○ | ◎ |
| Example 3 | 235 | 12 | 6 | 3 | PP spunbond SMS | 1.5 | 17 | Design roller | 157 | 0.6 | 41 | 70 | 59 | ◎ | ◎ | ○ | ◎ |
| Example 4 | 235 | 12 | 6 | 3.5 | PP spunbond SMS | 1.5 | 17 | Design roller | 157 | 0.6 | 45 | 77 | 58 | ◎ | ◎ | ◎ | ◎ |
| Example 5 | 310 | 8 | 5 | 3 | PP spunbond SMS | 1.5 | 17 | Design roller | 207 | 0.8 | 41 | 69 | 59 | ◎ | ◎ | ○ | ◎ |
| Comparative example 1 | 235 | 12 | 6 | 4 | PP spunbond SMS | 1.5 | 17 | Comb gun | 157 | 0.5 | Insufficient elongation to measure | Insufficient elongation to measure | Insufficient elongation to measure | × | × | × | × |
| Comparative example 2 | 156 | 18 | 6 | 3 | PP spunbond SMS | 1.5 | 17 | Comb gun | 104 | 0.5 | Insufficient elongation to measure | Insufficient elongation the measure | Insufficient elongation to measure | Cannot be worn | × | × | × |
| Comparative example 3 | 620 | 8 | 10 | 3 | PP spunbond SMS | 1.5 | 17 | Comb gun | 413 | 1.5 | 40 | 122 | 33 | | ○ | △ | × |
| Comparative example 4 | 620 | 8 | 10 | 3 | PP spunbond SMS | 1.5 | 17 | Design roller | 413 | 1.6 | 83 | 141 | 59 | | ○ | △ | ○ |

14

The composite laminate body according to the present invention can be applied to any composite laminate body that is required to be provided with stretchable gather parts with excellent feel when wearing and is suitable for applications such as materials for the underwear, sportswear or sanitary goods such as disposable diapers for children and adults, and in particular, is suitable for use as a disposable diaper.

DESCRIPTION OF CODES

10      Composite laminate body
11      Test material
100     Disposable diaper prototype
101     Gather part

**Claims**

1.  A composite laminate body, comprising:

    a plurality of polyurethane elastic fibers arranged in parallel in one direction, interposed between two sheets of cloth; and
    resin for bonding the elastic fibers and the two sheets of cloth, provided in a plurality of strands arranged to be parallel in one direction that intersects with the elastic fibers;
    wherein the two sheets of cloth and the elastic fibers are separated between locations where adjacent resin strands and elastic fibers intersect in the longitudinal direction of the elastic fibers;
    the fineness of the elastic fibers is 350 dtex or less;
    a ratio of fineness of the elastic fibers with regard to the fineness of the fibers that compose the cloths is 0.5 or higher and 300 or lower; and
    when two cycles of 130% elongation and return are performed according to the test method defined in the description, the following Formula 1 is satisfied, where

    (A) is the return stress at 30% elongation in the second cycle, and
    (B) is the return stress at 100% elongation in the second cycle.

    $$[\text{Formula 1}]\ (A)/(B) \times 100\% \geq 50\%$$

2.  The composite laminate body according to claim 1, wherein the composite laminate body has an elongation of 150% or more when a forward stress during the first cycle elongation is 1000 cN/5 cm width.

3.  The composite laminate body according to claim 1 or 2, wherein when the composite laminate body is in a condition of being stretched to a maximum in the direction that the elastic fibers extend, and the color of the outer surface of the cloth is measured using a spectrocolorimeter from outside of the composite laminate body in the direction orthogonal to the elastic fibers, in the following formula using the $L^*$ value, $a^*$ value, $b^*$ value at a point where the $L^*$ value is a maximum ($L^*1$, $a^*1$, $b^*1$) and $L^*$ value, $a^*$ value, $b^*$ value at a point where the $L^*$ value is a minimum ($L^*2$, $a^*2$, $b^*2$), the color difference variation defined by $\Delta E^*v$ is 1.0 or lower.

    $$\Delta E^*v = \sqrt{[(L^*1-L^*2)2+(a^*1-a^*2)2+(b^*1-b^*2)2]}$$

4.  The composite laminate body according to any one of claims 1 to 3, wherein the thickness of the composite laminate body at a location where the resin that extends in one direction intersects with the elastic fibers is 0.1 mm or higher and 2.0 mm or lower.

5.  The composite laminate body according to any one of claims 1 to 4, wherein the maximum value for the thickness of the composite laminate body between locations where the resin that extends in one direction intersects with the elastic fibers and that are adjacent in the longitudinal direction of the elastic fibers is 1 mm or higher and 20 mm or lower.

**6.** The composite laminate body according to any one of claims 1 to 5, wherein the width of one strand of resin that extends in one direction is 0.2 mm or more and 10 mm or less, in a condition where the composite laminate body is extended to a maximum in the direction that the elastic fibers extend.

**7.** The composite laminate body according to any one of claims 1 to 6, wherein the interval between strands of resin that extends in one direction is 1 mm or more and 20 mm or less, in a condition where the composite laminate body is extended to a maximum in the direction that the elastic fibers extend.

**8.** The composite laminate body according to any one of claims 1 to 7, wherein the thermal softening point of the elastic fiber is 100°C or higher and 240°C or lower.

**9.** The composite laminate body according to any one of claims 1 to 8, wherein the resin that extends in one direction includes a same component as a component that constitutes the cloths and/or the elastic fibers.

**10.** A disposable diaper, using the composite laminate body according to any one of claims 1 to 9 as a gather part.

**Patentansprüche**

**1.** Verbundlaminatkörper, umfassend:

eine Vielzahl von elastischen Polyurethanfasern, die in einer Richtung parallel angeordnet sind, angeordnet zwischen zwei Lagen aus Gewebe;
Harz zum Verbinden der elastischen Fasern und der beiden Lagen aus Gewebe, bereitgestellt in einer Vielzahl von Strängen, die in einer Richtung, die die elastischen Fasern schneidet, parallel angeordnet sind;
wobei die beiden Lagen aus Gewebe und die elastischen Fasern zwischen Orten, an denen sich benachbarte Harzstränge und elastische Fasern in der Längsrichtung der elastischen Fasern schneiden, getrennt sind;
die Feinheit der elastischen Fasern 350 dtex oder weniger beträgt;
ein Verhältnis der Feinheit der elastischen Fasern bezogen auf die Feinheit der Fasern, die die Gewebe bilden, 0,5 oder größer und 300 oder kleiner ist; und
wenn zwei Zyklen von 130 % Dehnung und Rückkehr gemäß dem in der Beschreibung definierten Prüfverfahren durchgeführt werden, die nachstehende Formel 1 erfüllt ist, wobei

(A) die Rückführspannung bei 30 % Dehnung bei dem zweiten Zyklus ist und
(B) die Rückführspannung bei 100 % Dehnung bei dem zweiten Zyklus ist.

$$[\text{Formel 1}] \quad (A)/(B) \times 100\ \% \geq 50\ \%$$

**2.** Verbundlaminatkörper gemäß Anspruch 1, wobei der Verbundlaminatkörper eine Dehnung von 150 % oder mehr aufweist, wenn eine Vorwärtsspannung während des ersten Dehungszyklus 1000 cN/5 cm Breite beträgt.

**3.** Verbundlaminatkörper gemäß Anspruch 1 oder 2, wobei, wenn der Verbundlaminatkörper in einem Zustand einer Dehnung bis zu einem Maximum in der Richtung, in der die elastischen Fasern verlaufen, vorliegt, und die Farbe der Außenoberfläche des Gewebes unter Verwendung eines Spektrokolorimeters von außerhalb des Verbundlaminatkörper in der Richtung senkrecht zu den elastischen Fasern gemessen wird, in der nachstehenden Formel unter Verwendung des L*-Werts, a*-Werts, b*-Werts an einem Punkt, bei dem der L*-Wert ein Maximum ist (L*1, a*1, b*1) und des L*-Werts, a*-Werts, b*-Werts an einem Punkt, bei dem der L*-Wert ein Minimum ist (L*2, a*2, b*2), die durch ΔE*v definierte Variation des Farbunterschieds 1,0 oder kleiner ist.

$$\Delta E^*v = \sqrt{[(L^*1-L^*2)2+(a^*1-a^*2)2+(b^*1-b^*2)2]}$$

**4.** Verbundlaminatkörper gemäß einem der Ansprüche 1 bis 3, wobei die Dicke des Verbundlaminatkörpers an einer Stelle, bei der das Harz, das in einer Richtung verläuft, die elastischen Fasern schneidet, 0,1 mm oder größer und 2,0 mm oder kleiner ist.

**5.** Verbundlaminatkörper gemäß einem der Ansprüche 1 bis 4, wobei der Maximalwert der Dicke des Verbundlaminatkörpers zwischen Stellen, an denen das Harz, das in einer Richtung verläuft, die elastischen Fasern schneidet, und die in der Längsrichtung der elastischen Fasern benachbart sind, 1 mm oder größer und 20 mm oder kleiner ist.

6. Verbundlaminatkörper gemäß einem der Ansprüche 1 bis 5, wobei die Breite eines Strangs von Harz, der in eine Richtung verläuft, in einem Zustand, bei dem der Verbundlaminatkörper in der Richtung, in der die elastischen Fasern verlaufen, auf ein Maximum gedehnt ist, 0,2 mm oder mehr und 10 mm oder weniger beträgt.

7. Verbundlaminatkörper gemäß einem der Ansprüche 1 bis 6, wobei der Abstand zwischen Strängen von Harz, die in einer Richtung verlaufen, in einem Zustand, bei dem der Verbundlaminatkörper in der Richtung, in der die elastischen Fasern verlaufen, auf ein Maximum gedehnt ist, 1 mm oder mehr und 20 mm oder weniger beträgt.

8. Verbundlaminatkörper gemäß einem der Ansprüche 1 bis 7, wobei der thermische Erweichungspunkt der elastischen Faser 100 °C oder höher und 240 °C oder niedriger liegt.

9. Verbundlaminatkörper gemäß einem der Ansprüche 1 bis 8, wobei das Harz, das in einer Richtung verläuft, eine gleiche Komponente enthält wie eine Komponente, die die Gewebe und/oder die elastischen Fasern bildet.

10. Einwegwindel mit dem Verbundlaminatkörper gemäß einem der Ansprüche 1 bis 9 als ein Sammelteil.

**Revendications**

1. Corps stratifié composite, comprenant :

    une pluralité de fibres élastiques de polyuréthane agencées en parallèle dans une direction, interposées entre deux feuilles de tissu ; et
    une résine pour la liaison des fibres élastiques et des deux feuilles de tissu, fournie en une pluralité de brins agencés de manière à être parallèles dans une direction qui croise les fibres élastiques ;
    dans lequel les deux feuilles de tissu et les fibres élastiques sont séparées entre des emplacements où des brins de résine et des fibres élastiques adjacents se croisent dans la direction longitudinale des fibres élastiques ;
    la finesse des fibres élastiques est de 350 dtex ou moins ;
    un rapport de finesse des fibres élastiques par rapport à la finesse des fibres qui composent les tissus est de 0,5 ou plus et de 300 ou moins ; et
    lorsque deux cycles de 130 % d'allongement et de retour sont effectués selon le procédé de test défini dans la description, la formule 1 suivante est satisfaite, où

        (A) correspond à la contrainte de retour à 30 % d'allongement dans le deuxième cycle, et
        (B) correspond à la contrainte de retour à 100 % d'allongement dans le deuxième cycle.

$$[\text{Formule 1}] \quad (A)/(B) \times 100\ \% \geq 50\ \%$$

2. Corps stratifié composite selon la revendication 1, le corps stratifié composite ayant un allongement de 150 % ou plus lorsqu'une contrainte directe pendant l'allongement de premier cycle est de 1 000 cN/5 cm de largeur.

3. Corps stratifié composite selon la revendication 1 ou 2, dans lequel lorsque le corps stratifié composite est dans une condition où il est étiré à un maximum dans la direction dans laquelle les fibres élastiques s'étendent, et la couleur de la surface extérieure du tissu est mesurée à l'aide d'un spectrocolorimètre depuis l'extérieur du corps stratifié composite dans la direction orthogonale aux fibres élastiques, dans la formule suivante utilisant la valeur L*, la valeur a*, la valeur b* à un point où la valeur L* est un maximum (L*1, a*1, b*1) et la valeur L*, la valeur a*, la valeur b* à un point où la valeur L* est un minimum (L*2, a*2, b*2), la variation de la différence de couleur définie par ΔE*v est de 1,0 ou moins.

$$\Delta E^*v = \sqrt{[(L^*1-L^*2)2+(a^*1-a^*2)2+(b^*1-b^*2)2]}$$

4. Corps stratifié composite selon l'une quelconque des revendications 1 à 3, dans lequel l'épaisseur du corps stratifié composite à un emplacement où la résine qui s'étend dans une direction croise les fibres élastiques est de 0,1 mm ou plus et de 2,0 mm ou moins.

5. Corps stratifié composite selon l'une quelconque des revendications 1 à 4, dans lequel la valeur maximale pour l'épaisseur du corps stratifié composite entre des emplacements où la résine qui s'étend dans une direction croise les fibres élastiques et qui sont adjacents dans la direction longitudinale des fibres élastiques est de 1 mm ou plus et de 20 mm ou moins.

6. Corps stratifié composite selon l'une quelconque des revendications 1 à 5, dans lequel la largeur d'un brin de résine qui s'étend dans une direction est de 0,2 mm ou plus et de 10 mm ou moins, dans une condition où le corps stratifié composite est étendu à un maximum dans la direction dans laquelle les fibres élastiques s'étendent.

7. Corps stratifié composite selon l'une quelconque des revendications 1 à 6, dans lequel l'intervalle entre des brins de résine qui s'étendent dans une direction est de 1 mm ou plus et de 20 mm ou moins, dans une condition où le corps stratifié composite est étendu à un maximum dans la direction dans laquelle les fibres élastiques s'étendent.

8. Corps stratifié composite selon l'une quelconque des revendications 1 à 7, dans lequel le point de ramollissement thermique de la fibre élastique est supérieur ou égal à 100 °C et inférieur ou égal à 240 °C.

9. Corps stratifié composite selon l'une quelconque des revendications 1 à 8, dans lequel la résine qui s'étend dans une direction inclut un même composant qu'un composant qui constitue les tissus et/ou les fibres élastiques.

10. Couche jetable, utilisant le corps stratifié composite selon l'une quelconque des revendications 1 à 9 comme pièce de rassemblement.

FIG. 1

FIG. 2

FIG. 3

FIG. 4

*10*

## FIG. 5A

## FIG. 5B

## FIG. 5C

*11*

## FIG. 5D

FIG. 6

**EP 3 720 403 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014207973 A
- JP 2005320636 A
- JP 2013202056 A
- JP 2017185336 A
- JP 2014076077 A